# EUROPEAN PATENT SPECIFICATION

(11) **EP 2 914 107 B1**
(45) Date of publication and mention of the grant of the patent: **29.04.2020**
(21) Application number: 13785852.8
(22) Date of filing: 31.10.2013
(51) Int. Cl.: A01N 25/30, A01N 43/54, A61K 9/00, A61K 47/24, A01P 7/00

(54) **TOPICAL PARASITICIDAL FORMULATION FOR USE AGAINST FLY STRIKE MYIASIS IN SHEEP**
TOPISCHE PARASITIZIDE FORMULIERUNG ZUR VERWENDUNG GEGEN FLYSTRIKE MYIASIS BEI SHAFE
FORMULATION PARASITICIDE TOPIQUE Á UTILISER CONTRE LA MYIOSE DE MOUCHE CHEZ L'OVIN

(30) Priority: 01.11.2012 AU 2012904792
(43) Date of publication of application: 09.09.2015
(73) Proprietor: Intervet International B.V., 5831 AN Boxmeer (NL)
(72) Inventor: DE ASIS, Emmanuel Gonzales, Seven Hills New South Wales 2147 (AU)
(74) Representative: Intervet International B.V.
(86) International application number: PCT/EP2013/072765
(87) International publication number: WO 2014/068028

(56) References cited:
- EP-A1- 0 521 607
- WO-A1-2010/130983
- WO-A1-2011/035288
- WO-A2-2006/100489
- US-A1- 2012 171 313
- "Insect Repellent for Dogs", , 1 May 2007 (2007-05-01), pages 1-2, XP55091066, Retrieved from the Internet: URL:http://www.gnpd.com/sinatra/recordpage /710243/from_search/M1xPagPe7S/ [retrieved on 2013-12-02]
- AKIMOTO T ET AL: "Polymeric transdermal drug penetration enhancer - The enhancing effect of oligodimethylsiloxane containing a glucopyranosyl end group", JOURNAL OF CONTROLLED RELEASE, ELSEVIER, AMSTERDAM, NL, vol. 77, no. 1-2, 9 November 2001 (2001-11-09), pages 49-57, XP004311662, ISSN: 0168-3659, DOI: 10.1016/S0168-3659(01)00455-2

## Description

### Field of the Invention

The present invention relates to a topical formulation for use in fly strike myiasis in sheep.

### Background Art

Sheep and other domesticated livestock are subject to infestation by a wide range of ectoparasites such as lice, blow-fly larvae, ticks, head flies, keds and sheep scab. Fly strike (myiasis) in sheep is a significant problem causing significant suffering to the animal and loss of production in infected livestock. At certain times of the year when blow flies are active, the adult blow fly lays eggs on the sheep. When these eggs hatch, the fly enters a series of larval stages and, during some of these stages, the larvae feed on the flesh of the infected sheep. This is known as fly strike or myiasis. Fly strike is caused by a number of species of blow fly, including *Lucilia cuprina, L. sericata, Chrysomia rufifacies,* and *Calliphora stygia.*

Traditionally infestations of ectoparasites on animals have been prevented or treated by applying a parasiticide to the entire body of the animal by either dipping the whole animal in a bath containing the parasiticide or by spraying a formulation containing the parasiticide over the entire body surface of the animal. More recently, certain formulations of some parasiticides have been developed that can be applied by localised application (so-called "pour-on" or "spray-on" application) to prevent or control ectoparasites on an animal. By "localised application" it is meant that the formulation is only applied to a minor portion of the outer surface of the animal, generally as a line or spot on the animal's back. External parasites on an animal are typically more prevalent at the rear, crutch or underside of the animal. In order for formulations applied by localised application to be able to control ectoparasites over the entire body of the animal, the active ingredient must be able to spread over the entire body of the animal. For example, pour-on formulations are typically applied to the back of an animal, while the parasites infesting the animal are usually located at the rear, crutch or underside of the animal.

A wide variety of parasiticides have been used to treat and/or prevent various ectoparasites on livestock. For example, agents that act to kill parasites on contact with, or ingestion by, the parasite have been used to treat and/or prevent fly strike on sheep. Another class of parasiticides effective to treat and/or prevent ectoparasite infestations are the insect growth regulators (IGRs). IGRs disrupt the growth cycle of insects.

A variety of IGRs have been described in the published literature, including 4,6-diamino-2-(cyclopropylamino)-5-pyrimidinecarbonitrile (dicyclanil). Several different formulations containing dicyclanil for application by a variety of different means are commercially available. These different types of formulation include non-aqueous and aqueous formulations.

WO2011/035288 discloses aqueous suspension pour-on or spray-on formulations comprising dicyclanil involving sodium lignosulphonate to prevent crystal growth of dicyclanil when suspended in water.

The use of organo-silicone surfactants in compositions for parasite control has been described in the following documents:
WO2010/130983 discloses the use of an ectoparasiticidal composition comprising a low viscosity and a high viscosity siloxane (dimethicone) and optionally an essential oil for the control of head lice in human beings.

US2012/0171313 discloses the use of an ectoparasiticidal composition for spraying or fogging to a parasite infested area including a neem oil, a siloxane as carrier and an essential oil as masking agent.

A number of documents disclose organosilicone compounds as penetration enhancing component in transdermal formulations for administration on human skin (WO2006/100489, EP0521607 Akimoto T et al: "Polymeric transdermal drug penetration enhancer - The enhancing effect of oligodimethylsiloxane containing a glucopyranosyl end group" Journal of controlled release, Vol 77, 1-2, (2001), pages 49-57).

Insect repellent spot-on products for dogs, that are based on essential oil and include the organosilicone compound dimethicone as component are commercially available (Spot-on Margosa 150).

In view of the significant problem of parasite infestation of animals, there is a continuing need to develop and improve formulations for preventing and treating parasite infestations.

### Disclosure of the Invention

The current invention provides a topical formulation for use in the prevention or treatment of fly strike myiasis in sheep, wherein the formulation is topically applied as a pour-on or spray-on technique, the formulation comprising 20 g/L to 60 g/L dicyclanil in suspension, 20 g/L to 200 g/L of 3-(3-hydroxypropyl)- heptamethylsiloxane, ethoxylated, acetate surfactant and water.

### Detailed description

The inventor of the present invention has surprisingly found that a topical formulation as used in the invention has greater spreading, wetting and infiltration (i.e. penetration into wool or fleece) capacity than similar formulations containing other surfactants or not containing a surfactant. The spreading, wetting and infiltration properties of the formulation allow the parasiticide to readily spread over the entire body of an animal to control ectoparasites over the entire body of the animal.

Further, when the formulation is applied to the wool or fleece of an animal, the wetting, spreading and infiltration properties of the formulation promote rapid uptake of the formulation, and therefore the parasiticide, into the wool or fleece of the animal. The rapid uptake of the parasiticide improves the rain fastness of the formulation, i.e. reduces the amount of the parasiticide washed off the animal in the event that the animal is exposed to rain shortly after the formulation has been topically applied to the animal.

The formulation is an aqueous formulation. The topically acceptable liquid carrier is therefore an aqueous carrier. Typically, the formulation comprises about 300 g/L or more, more typically about 500 g/L or more, of water. Aqueous formulations are preferable as such formulations are generally easier and safer to handle than formulations comprising a large proportion of an organic solvent.

The formulation is to be used for the prevention or treatment of myiasis. Formulations of the present invention comprising dicyclanil, can provide continuing protection against fly strike for an extended period, e.g. up to 14 weeks.

The formulation may be provided as a concentrate intended to be diluted prior to application to an animal, or may be provided as a ready-to-use formulation. The formulation when applied to the animal typically contains an effective amount of the parasiticide such that a convenient volume of the formulation is required to provide an effective dose of the parasiticide to prevent or treat an infestation of ectoparasites on the animal. A convenient volume of a formulation intended for localised topical application, e.g. in a line or lines on the back of the animal, is generally less than about 3 mL per kg body weight. A person skilled in the art could readily determine suitable concentrations of the parasiticide based on the amount of the parasiticide required to provide an effective dose and the volume of the formulation to be topically applied to the animal.

The formulation of the present invention comprise dicyclanil at a concentration of from about 20 g/L to about 60 g/L. For example, the formulation may comprise dicyclanil in a concentration of from about 30 g/L to about 60 g/L, e.g. from about 20 g/L to about 50 g/L, from about 33 g/L to about 55 g/L, or from about 33 g/L to about 50 g/L.

The formulation of the present invention comprises the organo-silicone surfactant of the current invention in an amount of about 20 g/L to about 200 g/L, e.g. about 20 g/L to about 170 g/L, about 50 g/L to about 200 g/L, about 50 g/L to about 170 g/L, about 70 to about 150 g/L or about 80 to about 120 g/L.

As used herein, the term "organo-silicone surfactant" refers to any surface active compound comprising at least one Si-C bond.

**Table 1**

| **PRODUCT NAME** | **MANUFACTURER /DISTRIBUTOR** | **ADJUVANT CATEGORY** | **PRINCIPAL FUNCTIONING AGENTS** |
|---|---|---|---|
| SYLGARD 309 (also sold under the name XIAMETER OFX 309) | Wilbur-Ellis Company; Dow Corning Australia Pty Ltd | Organo-Silicone Surfactant | Organosilicone surfactant |

The organo-silicone surfactant used in the invention is 3-(3-hydroxypropyl)-heptamethyltrisiloxane, ethoxylated, acetate (i.e. 2-[acetoxy(polyethyleneoxy)proyl]heptamethyltrisiloxane).This surfactant is available commercially under the trade names Sylgard 309 (Dow Corning Australia Pty Ltd), The molecular formula of 3-(3-hydroxypropyl)-heptamethyltrisiloxane, ethoxylated, acetate is (C₂H₄O)ₙC₁₂H₃₀O₄Si₃, wherein n is an integer from 1 to 100, preferably from 1 to 50, more preferably from 1 to 15, most preferably from 3 to 10.

The inventors of the present invention have surprisingly found that the formulation of the present invention spreads more effectively over the skin of an animal than similar formulations not containing about 20 g/L or more of organo-silicone surfactant.

The spreading, wetting and infiltration properties of the formulation contribute to the efficacy of the formulation in preventing and controlling ectoparasites on an animal. To minimise handling of an animal, it is preferable to be able to topically apply a parasiticidal formulation to the back of the animal or other localised portion of the skin or fleece of the animal, rather than apply the formulation to the entire body of the animal. However, ectoparasites on an animal are typically more prevalent in the rear, crutch or underside of the animal. For example, flystrike on sheep is typically most severe at the rear and crutch of the animal. The ability of a parasiticidal formulation to move through the hair, wool or fleece of an animal to control ectoparasites over the entire body of the animal is a significant factor affecting the efficacy of a parasitical formulation applied by localised topical application (e.g. to the back, or part of the back, of the animal) to control ectoparasites on the animal.

In addition to facilitating the spreading, wetting and penetration of the formulation, the organo-silicone surfactant in the formulation of the present invention may also assist in stabilising the formulation and, for parasiticides that are sparingly soluble in water, may assist in suspending the parasiticide in an aqueous carrier.

The topically acceptable liquid carrier is water. Typically, the formulation comprises about 300 g/L or more of water, e.g. about 500 g/L or more, about 600 g/L or more, 650 g/L or more or 700 g/L or more, of water.

The formulation may contain one or more other surfactants in addition to one or more organo-silicone surfactants. The formulation may, for example, comprise a wetting agent. When the formulation comprises a water insoluble parasiticide suspended in an aqueous carrier, the formulation typically comprises one or more wetting agents to facilitate the wetting of the parasiticide during manufacture of the formulation. The formulation may also comprise one or more additional surfactants to assist in the dispersal of the parasiticide in the formulation or to assist in the spreading of the formulation on the animal when the formulation is topically applied to the animal. The additional surfactant may be a non-ionic, an anionic or a cationic surfactant. Examples of non-ionic surfactants include sorbitan esters, polyoxyalkylated sorbitan esters including polyoxyalkylated sorbitan fatty acid esters, polyoxyalkylated alkyl ethers, polyoxyalkylated fatty alcohols (also known as fatty alcohol alkoxylates), polyoxyalkylated fatty acids, polyalkylene glycol esters, polyoxyalkylated vegetable oils, polyglycerol esters, copolymers of ethylene oxide and propylene oxide, fatty amine alkoxylates, alkylphenol alkoxylates, alkyl polysaccharides, polymeric surfactants. Examples of anionic surfactants include linear alkylbenzene sulphonates, alkylnaphthalene sulphonates, C₁₂ to C₁₆ alcohol sulphates, C₁₂ alkoxypolyethanoxy sulphates, alkyl phosphates and phosphonates, alkylated alkylphenols, alkylphenol polyalkylate phosphate esters and combinations thereof. Examples of cationic surfactants include benzalkonium, cetyltrimethylammonium chloride, cetyl pyridinium chloride, cetyl trimethylammonium bromide, tonzonium bromide and combinations thereof. In some embodiments, the formulation may comprise a combination of compatable non-ionic, anionic and/or cationic surfactants, e.g. one or more non-ionic surfactants in combination with one or more anionic surfactants.

The additional surfactant or surfactants may, for example, be present in an amount of about 10 g/L to about 450 g/L, about 50 g/L to about 400 g/L or about 200 g/L to about 400 g/L of the total formulation.

In some embodiments, the formulation of the present invention may further comprise one or more agents selected from water-miscible co-solvents, pH modifiers, buffers, preservatives, anti-foam agents, dyes, adjuvants, wetting agents, viscosity modifiers and humectants.

Typically the formulation comprises one or more of a buffer or a preservative to extend the shelf-life of the formulation.

The formulation may comprise one or more buffers or pH adjustors to adjust the pH of the formulation and maintain the pH of the formulation at a pH suitable to extend the shelf life of the formulation.

Suitable buffers will generally maintain the pH of the formulation in the range of about 4 to about 8, e.g. 5.5 to 7.5, 6 to 7.5, or 6.5 to 7.5. Suitable buffers include soluble monobasic and/or dibasic phosphates, such as sodium dihydrogen orthophosphate. Alternatively, a buffering system may be used where a combination of two or more buffers and/or pH modifying agents are combined to provide the desired pH range. The buffer may, for example, be present in the formulation in an amount of about 0.2 to about 20 g/L, for example, about 5 to about 10 g/L, based on the total formulation.

Suitable pH modifiers may be used independently or in conjunction with compatible buffers either as a separate additive or as part of a buffering system. Suitable pH modifiers include sodium hydroxide, potassium hydroxide, calcium hydroxide, sodium carbonate, potassium carbonate, ascorbic acid and citric acid.

The preservative may, for example, be an anti-oxidant, anti-microbial, free-radical scavenger or any other agent that extends the shelf-life of the formulation. For example, the preservative may be selected from formaldehyde, diazolidinyl urea, hydroxybenzoate derivatives including sodium methyl hydroxybenzoate and sodium propyl hydroxybenzoate, 1,2-benzisothiazolin-3-one, sodium hydroxymethylglycinate, benzoic acid, sodium benzoate, sodium propionate, sorbic acid, benzyl alcohol, bronopol, chlorbutol, phenoxyethanol, o-phenoxyethanol, chlorhexidine salts, phenylmercuric salts, thiomersal, chlorocresol, cresol, phenol, benzalkonium chloride, cetrimide, alpha-tocopherol, ascorbic acid, sodium ascorbate, butylated hydroxyanisole, butylated hydroxytoluene or sodium metabisulfite, or a combination thereof. A suitable anti-microbial for use in the formulation is 1,2-benzisothiazolin-3-one.

The preservative may, for example, be present in the formulation in an amount of about 0.5 g/L to about 10 g/L based on the total formulation. In some embodiments, the preservative is present in an amount of about 1.0 g/L to about 5 g/L based on the total formulation.

The formulation may comprise one or more anti-foam agents to prevent foaming of the formulation during manufacture of the formulation and during application of the formulation to an animal. Suitable anti-foam agents include, for example, compounded silicone fluid (Anti-Foam A) or Gensil 2030 (Anti-Foam C). The anti-foam agent may be present in the formulation in, for example, about 0.1 g/L to about 5 g/L, e.g., in an amount of about 0.5 g/L to about 2 g/L or about 0.5 g/L to about 1.5 g/L, based on the total formulation.

The formulation typically comprises a colouring agent or dye. Colouring agents enable treated animals to be readily distinguished from untreated animals. The colouring agent may be dissolved, suspended or dispersed in the formulation. The nature of the colouring agent is unimportant and a wide variety of suitable dyes and pigments will be known to a person skilled in the art. The colouring agent may be soluble or insoluble in water. Generally, however, the colouring agent will be biodegradable so as to fade and not permanently mark the skin or fleece, or scourable so that it can be removed from wool during processing. Examples of suitable colouring agents include: Toluidine Red (CI Pigment Red 3), FD&C Brilliant Blue No. 1 (Brilliant Blue FCF, Hexacol Brilliant Blue), Fast Scarlet Pigment 3610 (Dispers Scarlet FK3610), Luconyl Green FK872, Fluoresceine LT, Tartrazine, Carmine Dispersion R1276 and C.I. Food Red (Carmosine, CI 14720).

The formulation is a suspension of dicylanil.

The formulation will typically include a thickener to assist in maintaining the parasiticide in suspension. The thickener may be, for example, acacia, agar, alginic acid, aluminium magnesium silicate, aluminium monostearate, bentonite, carbomers, carmellose, carrageenan, cellulose, ceratonia, cetostearyl alcohol, cetyl alcohol, ethylcellulose, gellan gum, xanthan gum, guaraprolose, hydroxypropyl cellulose, hyetellose, hymetellose, hyprolose, hypromellose or hydroxypropyl methylcellulose, methylcellulose, microcrystalline cellulose, polyethylene oxide or polyethylene glycol, polypropylene glycol, polyvinyl acetate, polyvinyl alcohol, povidone, polyvinyl pyrollidine, silicas, stearyl alcohol and tragacanth, or a combination thereof. In some embodiments, the thickener is a polyethylene glycol, such as PEG300, polypropylene glycol, xanthan gum, microcrystalline cellulose, polyvinyl pyrrolidine or hydroxypropyl cellulose, or a combination thereof.

The thickener may, for example, be present in amounts of about 0.5 g/L to about 15 g/L, about 1 g/L to about 10 g/L or about 2 g/L to about 8 g/L based on the total formulation.

The formulation may comprise a humectant to retain moisture in the formulation and prevent the formulation from drying out during storage. The humectant may be, for example, calcium saccharate, calcium stearoyl-lactylate, ceratonia, cetostearyl alcohol, diethylene glycol monopalmitostearate, dipropylene glycol, cyclodextrins, ethylenediamine, ethylene glycol monopalmitostearate, gentisic acid ethanolamide, glycerine, glyceryl monostearate, hexylene glycol, maleic acid, monoglyceride citrate, potassium metaphosphate, propylene glycol, sodium stearoyl-lactylate, sorbitol, sucrose esters or trehalose, or a mixture thereof.

The humectant may, for example, be present in the formulation in amounts of about 10 g/L to 100 g/L, about 20 g/L to about 80 g/L, or about 25 g/L to about 75 g/L, based on the total formulation.

In some embodiments, the formulation comprises two or more parasiticides. The formulation may, in some embodiments, comprise one or more other active agents in addition to dicyclanil.

The formulation is topically applied to sheep to control fly strike myiasis on the animal.

The formulation may be applied to the animal by localised topical application.

The animal is a sheep.

An effective amount of the formulation to prevent or treat fly strike on sheep is an amount containing about 20 to about 200 mg/kg of dicyclanil relative to the total body weight of the sheep.

The formulation is topically applied to less than about 50 % of the outer surface of the animal, more preferably to less than about 35 % of the outer surface of the animal and most preferably to less than about 20 % of the outer surface of the animal.

In a preferred embodiment, the formulation is applied to the animal by localised topical application.

In some embodiments, the localised area to which the formulation is applied topically is to the area infested with parasites or likely to be infested with parasites. More typically, the formulation is applied in a line or multiple lines, or in a spot, on the animal's back.

The formulation is topically applied to sheep susceptible to an infestation of fly strike myiasis to prevent an infestation of ectoparasites on the animal. The formulation is topically applied to sheep having an infestation of fly strike myiasis to treat or control the ectoparasites infestation on the animal by eliminating the ectoparasites from the animal, reducing the severity of the ectoparasite infestation on the animal, or retarding the progression or spread of the ectoparasite infestation on the animal.

The formulation used in the present invention is applied to the sheep by a spraying technique, or a pour-on technique. Suitable formulations may be applied in a liquid form or an aerosol form. The aerosol form may use a liquid or a gas as a propellant. In an embodiment, the formulation for use according to the invention further comprises a humectant.

In another embodiment, the formulation for use in the current invention further comprises a wetting agent or other surfactant.

In another embodiment, the formulation for use in the current invention further comprises a thickener.

In an embodiment, the formulation for use in the current invention further comprises at least one of a buffer, an anti-microbial agent, a pH modifier, an anti-foam agent and/or a dye.

An embodiment of a formulation for use in the present invention is as follows:

| **Ingredient** | **g/L*** |
|---|---|
| Parasiticide (e.g. Dicyclanil) | 33.33 |
| Organo-silicone surfactant (e.g. Sylgard 309) | 100.00 |
| Purified Water - Pharmacopeial Grade | qs to 1L |
| Humectant (e.g. Propylene Glycol) | 75.98 |
| Anti-foam (e.g. Compounded Silicone Fluid (e.g. Antifoam A Compound, Food Grade) or Non - Ionic Aqueous Emulsion Containing a Polydimethylsiloxane (e.g. Gensil 2030)) | 1.50 |
| Colouring agent (e.g. Dispers Scarlet FK 3610) | 0.30 |
| Thickener (e.g. Magnesium Aluminum Silicate (e.g.Veegum Regular) and Xanthan Gum (e.g. Kelzan S)) | 7.58 |
| Preservative (e.g. 1,2 - Benzisothiazolin - 3 - one (e.g. Proxel GXL)) | 2.50 |
| pH modifier (e.g. Sodium Hydroxide) | 0.40 |
| Wetting agents and other surfactants (e.g. Polyoxyethylene 20 Sorbitan Monooleate (e.g. Ecoteric T20); Alkylnapthalene Sulfonate Condensate, Sodium Salt (e.g. Morwet D425); Nonylphenol, Ethoxylated Blend (e.g. Teric 200); and Nonylphenol Polyethoxylate Phosphate Ester (e.g. Gafac RE 610)) | 61.00 |

| | |
|---|---|
| Notes: * Concentrations provided are approximate and are intended to be within +/- 10% | |

The formulation of the present invention may be prepared by any suitable technique. For example, an aqueous formulation comprising a water insoluble parasiticide may be prepared by first preparing a concentrate of the parasiticide, milling the concentrate, mixing the milled concentrate with a water phase, adjusting the pH of the resultant mixture to about 6.5 to about 7.5, and then adding the organo-silicone surfactant. The concentrate may, for example, be prepared by mixing the parasiticide, wetting agent, humectant, colouring agent and water. The water phase may comprise water, thickener, antifoam, preservative, buffer and pH adjuster.

### Examples

### Example 1: Comparative Example

A formulation comprising 50 g/L dicyclanil and 30 g/L of the surfactant TWEEN 80 was prepared comprising similar excipients in similar amounts to a commercially available topical formulation containing a different parasiticide. The formulation was then tested for efficacy in the control of flystrike. The formulation comprised the components listed below.

| **Ingredients** | **g/L** |
|---|---|
| Propylene Glycol | 60.00 |
| Xanthan Gum (Keltrol F) | 4.00 |
| Polyoxyethylene Sorbitan Monooleate (Tween 80) | 30.00 |
| Methyl Paraben | 1.80 |
| Propyl Paraben | 0.20 |
| Citric Acid | 1.80 |
| Disodium Hydrogen Phosphate | 6.65 |
| Dispers Scarlet 3610 | 0.30 |
| Phenol Sulfonic Acid Formaldehyde Concentrate (Tamol PP) | 3.00 |
| Simethicone (Antifoam A) | 0.65 |
| Simethicone (Antifoam C) | 0.35 |
| Dicyclanil | 50.00 |
| Deionised (DI) Water | q.s |

The above formulation was used in an Implant Trial Study to determine its efficacy against fly strike on sheep. In the Implant Trial Study the formulation was topically applied to the back, crutch and rump areas of the sheep at a dose rate of 52.5 mg to 105 mg dicyclanil per kg body weight. The study showed the formulation lacked sufficient efficacy and the study was terminated after 62 days.

Further tests showed that the formulation appeared beady on contact with wool. Various alternative formulations containing different surfactants or liquid lanolin were tested to assess the wetting and spreading properties. Amongst the formulations tested were formulations containing the surfactants Teric BL8, Termul 5030, Teric 200, Teric 169, Span 85 and Sylgard 309 at a concentration of 100 to 150 g/L. The resulting formulations were evaluated by applying 0.5 to 1.0 ml of each formulation to raw wool. The extent of wetting and spreading was observed visually. The formulation containing the organo-silicone surfactant, Sylgard 309, had greater spreading and wetting properties when applied to wool than the formulations containing other surfactants.

### Example 2: Evaluation of dicyclanil formulations against the Australian sheep blowfly larvae (Lucilia cuprina) using larval implants on sheep

### Formulation details

### Investigational Veterinary Product 1 (IVP 1):

### Formulation Components

| **Ingredients** | **g/L** |
|---|---|
| DI Water | 782.35 |
| Xanthan Gum (Keltrol F) | 2.00 |
| Propylene Glycol | 60.00 |
| Polyoxyethylene Sorbitan Monooleate (Tween 80) | 30.00 |
| Methyl Paraben | 1.80 |
| Propyl Paraben | 0.20 |
| Citric Acid | 0.70 |
| Disodium Hydrogen Phosphate | 6.65 |
| Disperse Scarlet 3610 | 0.30 |
| Phenol Sulfonic Acid Formaldehyde Conc. (Tamol PP) | 3.00 |
| Simethicone (Antifoam A) | 0.35 |
| Simethicone (Antifoam C) | 0.65 |
| Dicyclanil (98% Purity) | 35.00 |
| Sylgard 309 | 100.00 |

### Investigational Veterinary Product 2 (IVP 2): Formulation Components

| **Ingredients** | **g/L** |
|---|---|
| DI Water | 696.55 |
| Xanthan Gum (Keltrol F) | 2.00 |
| Propylene Glycol | 60.00 |
| Polyoxyethylene Sorbitan Monooleate (Tween 80) | 30.00 |
| Methyl Paraben | 1.80 |
| Propyl Paraben | 0.20 |
| Citric Acid | 1.50 |
| Disodium Hydrogen Phosphate | 6.65 |
| Disperse Scarlet 3610 | 0.30 |
| Phenol Sulfonic Acid Formaldehyde Conc. (Tamol PP) | 3.00 |
| Simethicone (Antifoam A) | 0.35 |
| Simethicone (Antifoam C) | 0.65 |
| Dicyclanil (98% Purity) | 35.00 |
| Solan E 50 (Liquid Lanolin - 50%) | 200.00 |

### Investigational Veterinary Product 3 (IVP 3): Formulation Components

| **Ingredients** | **g/L** |
|---|---|
| Disperse Scarlet 3610 | 0.30 |
| Dicyclanil (98% Purity) | 35.00 |
| Sylgard 309 | 100.00 |
| Dimethyl Acetate | 637.58 |
| Teric BL8 | 200.00 |

### Investigational Veterinary Product 4 (IVP 4): Formulation Components

| **Ingredients** | **g/L** |
|---|---|
| Dicyclanil (98% Purity)* | 35.00 |
| Fluilan (Lanolin) | 200.00 |
| 2 - Ethyl -1 Hexanol | 610.41 |
| Lanette 16 (Cetyl Alcohol) | 7.00 |
| Polyoxyethylene (20) Cetyl Ether | 10.00 |
| Butylatedhydroxyanisole | 0.10 |
| Colloidal Silicone Dioxide NF (Wacker HDK N20) | 10.00 |

IVP 1 and IVP 3 contain Sylgard 309. Sylgard 309 is an organo-silicone surfactant. IVP 1 and IVP 2 are aqueous formulations. IVP 3 and IVP 4 are non-aqueous formulations.

The investigational Veterinary Products were prepared by conventional formulation techniques. In summary, IVP 1 and IVP 2 were prepared by first preparing a concentrate of dicyclanil, milling the concentrate, and then mixing the milled concentrate with a water phase containing the remaining ingredients of the formulation. IVP 3 and IVP 4 were prepared by dissolving the dicyclanil in the organic solvent, and then mixing the resultant solution with the remaining components of the formulation; a further portion of organic solvent was added to obtain the desired concentration of dicyclanil.

### Results and discussion

One small paddock pen study was conducted over a period of five months. Forty sheep were randomly allocated to five treatment groups following ranking and blocking based on bodyweights. There was one spare sheep in each group. The sheep were group housed according to treatment and did not contact any other sheep during the study period.

The following treatments were evaluated in the study:

| **Treatment Group** | **Dicyclanil Concentration (g/L)** | **Dose Rate (mg/kg)** | **Number of Sheep Treated** | **Number of Sheep Receiving Implant** |
|---|---|---|---|---|
| **1. IVP 1 (aqueous Sylgard)** | 35 | 31.5-110.25 | 8 | 7 |
| **2. IVP 2 (aqueous Lanoline)-comparative example** | 35 | 31.5-110.25 | 8 | 7 |
| **3. IVP 3 (solvent Dmac) comparative example** | 35 | 31.5-110.25 | 8 | 7 |
| **4. IVP 4 (oil suspension) comparative example** | 35 | 31.5-110.25 | 8 | 7 |
| **5. Negative Control** | N/A | N/A | 8 | 7 |

On Day -1, all sheep were tagged, weighed and allocated. On Day 0, groups were treated with the appropriate treatments. In each of Groups 1 to 4, the formulation was topically applied to the back, crutch and rump areas of the sheep. Group 5 remained untreated.

After treatment, at 4, 8, 12, 16, 20 and 24 weeks after treatment (WAT), each sheep was implanted with first instar larvae of the laboratory bred strain of Australian sheep blowfly (*Lucilia cuprina*) and assessments were made at 24, 48 and 72 hours post-implant to assess the efficacy of the treatment in controlling larval populations and inhibiting larval growth and development.

Assessments included: number of larvae present, size of larvae, strike size and larvae appearance. Larvae surviving at 72 hours were removed from the treated sheep and placed into a labelled plastic container and maintained in the laboratory insectary on mutton to determine if they could complete their life cycle. The strikes were then clipped out and treated with sodium tetraborate powder to eliminate the viability of the strike and minimise animal discomfort. Negative control strikes were resolved after the 24 hour assessment.

IVP's 1, 2 and 3 showed consistently higher control of the strikes than IVP 4. IVP 4 (Oil suspension) never controlled all strikes at any time point during the trial and reached a maximum efficacy of 11/14 strikes controlled at 4, 8 and 12 WAT. After that the efficacy declined further to only 3/14 at 24 WAT.

The mean number of strikes controlled at 4 WAT was 14/14 for IVP 1 (Aqueous Sylgard) at 24 hours, IVP 2 (Aqueous Lanoline) reached 14/14 at 48 hours and IVP 3 (Solvent Dmac) was 14/14 at 72 hours post implant.

At 8 WAT the number of strikes controlled at 72 hours was 14/14 for IVP 2 and 13/14 for IVP 3 whilst IVP 1 reached 12/14 strikes controlled.

At 12 WAT, IVP 3 provided 14/14 strikes controlled at 24 hours, and IVP's 1 and 2 had controlled 13/14 of strikes at 72 hours. At 20 WAT the efficacy of IVP 2 declined sharply to 6/14 at 72 hours and the trend for this group continued at 24 WAT when only 4/14 of the strikes were controlled at 72 hours.

IVP 1 and IVP 3 maintained a higher efficacy level until the end of the study with 10/14 strikes controlled by both IVP 1 and IVP 3 at 20 WAT, and 9/14 and 8/14 strikes controlled at 24 WAT respectively.

All IVP groups with uncontrolled strikes had significantly fewer larvae and smaller strikes than the untreated control group at 24 hours post-implanting.

The best performing formulations were IVP 3 (Solvent Dmac) and IVP 1 (Aqueous Sylgard). Both IVP's showed a high level of efficacy until 16 WAT and the activity remained at moderate level until 24 WAT.

IVP 3 (as well as IVP 4) produced a strong odour at treatment however that was irritating to the eyes and nose. Therefore, IVP 1 was the only formulation that was safe to apply and provided a consistently high level of efficacy.

All larvae collected at 4 and 8 WAT from uncontrolled strikes in Groups 1, 3 and 4 failed to develop into pupae and died.

At 12 WAT larvae collected from Groups 1, 2 and 4 pupated in low numbers with 20% larvae pupating in Group 1 (1 pupa), 100% pupae developed from larvae collected in Group 2 (5 pupae) and 40% pupae formed in Group 4 (12 pupae).

At 16 WAT 12% pupated in Group 1 (3 pupae) and 57% larvae pupated in Group 4 (30 pupae).

The numbers of collected larvae as well as pupae increased at 20 WAT as the efficacy of the treatments declined with 50% of larvae developing into pupae in Group 1 (20 pupae), 49% in Group 2 (27 pupae) and 47% in Group 4 (28 pupae). In Group 3 (Dmac solvent) only 3 larvae were collected and no pupae were formed.

At 24 WAT there were larvae collected in all groups with 71% larvae progressed to pupal stage in Group 1 (27 pupae) and of that 1 adult fly emerged. 71% larvae developed into pupae in Group 2 (71 pupae) and 4 adults emerged. In Group 3, 72% larvae progressed to the pupal stage but no flies emerged. In Group 4, 29% larvae developed into pupae (28 pupae) and, of that, 4 adult flies emerged.

The data suggests that despite the fact the collected larvae appeared unaffected and were allowed to complete their life cycle in an untreated environment, their further development was inhibited as the pupal development was approximately 50% in all groups up to 20 WAT (with the exception of Group 2 in 12 WAT).

### Example 3: Evaluation of the activity of two experimental dicyclanil formulations against the Australian sheep blowfly larvae (Lucilia cuprina) using larval implants on sheep.

### Formulation details

### Investigational Veterinary Product 1 (IVP 5): Formulation Components

| **Formulation Components** | **g/L** |
|---|---|
| Purified Water | 739.938 |
| Morwet D 425 | 3.00 |
| Propylene Glycol | 75.98 |
| Polyoxyethylene Sorbitan Monooleate (Ecoteric T80) | 30.00 |
| Antifoam A | 0.85 |
| Antifoam C (Gensil 2030) | 0.65 |
| Disperse Scarlet 3610 | 0.30 |
| *Dicyclanil | 33.33 |
| Veegum | 5.80 |
| Kelzan S | 1.78 |
| Teric 200 | 22.4 |
| Gafac RE 610 | 5.60 |
| Formalin 37% | 0.30 |
| Sodium Hydroxide - 10% Solution | 56.0 |
| Sylgard 309 | 100.00 |

| | |
|---|---|
| * The amount of Dicyclanil was factored according to the purity of the batch used | |

### Investigational Veterinary Product 2 (IVP 6): Formulation Components

| **Formulation Components** | **g/L** |
|---|---|
| Purified Water | 783.52 |
| Acrylates/C10-30 Alkyl Acrylate Crosspolymer (Pemulen TR2) | 2.00 |
| Propylene Glycol | 60.00 |
| Polyoxyethylene Sorbitan Monooleate (Tween 80) | 30.00 |
| 1,2 - Benzisothiazolinone 3- one (BIT) (Proxel GXL) | 2.50 |
| Citric Acid | 0.70 |
| Disodium Hydrogen Phosphate | 6.65 |
| Disperse Scarlet 3610 | 0.30 |
| Alkylnaphthalene Sulfonate Condensate, Sodium Salt (Morwet D425) | 3.00 |
| Simethicone (Antifoam A) | 0.35 |
| Simethicone (Antifoam C) | 0.65 |
| *Dicyclanil | 33.33 |
| Sylgard 309 (Silicone Surfactant - Spreading Agent) | 100.00 |

| | |
|---|---|
| * The amount of Dicyclanil was factored according to the purity of the batch used IVP 5 was prepared by the following steps: Part A - Dicyclanil Concentrate 1. Transfer a portion of PURIFIED WATER to the manufacturing vessel. 2. Add with stirring MORWET D-425 and stir until dissolved. 3. Add with stirring a portion of PROPYLENE GLYCOL, ECOTERIC T80 and stir until dissolved, approximately 30 minutes. 4. Add with stirring a portion of ANTIFOAM A COMPOUND FOOD GRADE and GENSIL 2030 and stir until homogeneous. 5. Add with stirring a portion of PROXEL GXL. Continue stirring until homogeneous. 6. Add with stirring TOLUIDINE RED (i.e. DISPERS SCARLET FK 3610) to the manufacturing vessel. Rinse container with a portion of PURIFIED WATER and add to the batch. Continue stirring the batch slowly. 7. Add slowly with stirring the DICYCLANIL and stir for 30 minutes. Leave concentrate to age overnight before milling. Concentrate will turn thick & very dark pink. **Note:** When possible keep the batch stirring slowly overnight to avoid any settling and possible caking on the bottom of the tank. 8. Mix the concentrate for 15 minutes prior to milling. Mill Part A through a Dyno Mill containing 1.00 - 1.25 mm SEPR Zirconium Silicate beads to reduce the particle size of the Dicyclanil to 90% less than 10 µm. Once the required particle size has been achieved continue stirring slowly to prevent settling. 9. Rinse the Dyno Mill with a portion of PURIFIED WATER and add the rinsing to the batch. | |

### Part B - Water Phase

1. Melt Teric 200 in a hot water bath or oven overnight.
2. In a separate manufacturing vessel to Part A, add the following in sequence: portion of PURIFIED WATER and VEEGUM REGULAR. Stir for 10 minutes to disperse. Start heating to 70 - 80°C.
3. In a separate mixing vessel, premix a portion of PROPYLENE GLYCOL and KELZAN S. Stir to disperse the Kelzan S particles.
4. When the temperature of the main manufacturing vessel reaches 70 - 80°C, add the premix with stirring. Rinse the premix vessel with portion of PURIFIED WATER then add rinsings to batch. Continue mixing for 2 hours at 70 - 80°C to fully hydrate the gum.
5. In another mixing vessel, premix a portion of PROPYLENE GLYCOL and melted TERIC 200. Mix until homogeneous then transfer to the main manufacturing vessel. Rinse premix vessel with portion of PURIFIED WATER and add to batch. Continue mixing for 5 - 10 minutes. Start cooling the batch to 40°C.
6. When the temperature of the batch has reached 40°C, add in sequence to the main manufacturing vessel GAFAC RE 610 and portion of PROXEL GXL. Continue stirring for 5 minutes or until homogeneous.
7. Add slowly with stirring Part A DICYCLANIL CONCENTRATE, rinse the manufacturing vessel used for Part A with a portion of PURIFIED WATER and add to the batch, stir for 30 minutes.
8. In another mixing vessel, premix a portion of ANTIFOAM A COMPOUND FOOD GRADE to a portion of PURIFIED WATER. Mix until dissolved then add to main manufacturing vessel. Rinse with portion of PURIFIED WATER. Continue mixing for 10 minutes.
9. Take sample and test for pH. If required adjust pH to a pH range of 6.5 - 7.5. The pH can be lowered with CITRIC ACID SOLUTION or raised with portion of SODIUM HYDROXIDE Pellets dissolved in a portion of PURIFIED WATER if required. Continue stirring batch.
10. When the pH is correct, slowly add SYLGARD 309 and continue mixing for another 30 minutes.
11. Check the batch weight and volume.
12. Adjust according to planned batch weight and volume if too low (check aeration before any volume adjustment).
13. Transfer with a pump through a 100 micron monofilament GAF filter to a stainless steel holding tank in readiness for filling.
15. Fill into approved containers.

A similar method was used to prepare IVP 6.

### Results and discussion

One small paddock pen study was conducted over seven months. Twenty seven sheep were randomly allocated to three treatment groups following ranking and blocking based on bodyweights. There was one spare sheep in each group. The sheep were group housed according to treatment and did not contact any other sheep during the study period.

The following treatments were evaluated in the study:

| **Treatment Group** | **Dicyclanil Concentration (g/L)** | **Dose Rate (mg/kg)** | **Number of Sheep Treated** | **Number of Sheep Receiving Implants** |
|---|---|---|---|---|
| 1. IVP 5 | 33.33 | 30-105 | 9 | 8 |
| 2. IVP 6 | 33.33 | 30-105 | 9 | 8 |
| 3. Negative Control | NA | NA | 9 | 8 |

On Day 0, all sheep were tagged, weighed and allocated and Group 1 and Group 2 were treated with the appropriate treatments. For each of Groups 1 and 2, the formulation was topically applied to the back, crutch and rump areas of the sheep. Group 3 remained untreated.

At 28, 56, 84, 112, 140 and 168 days after treatment (DAT) each sheep was implanted with first instar larvae of the laboratory bred strain of Australian sheep blowfly (*Lucilia cuprina*) and assessments were made at 24, 48 and 72 hours post-implant to assess the efficacy of the treatment in controlling larval populations and inhibiting larval growth and development.

Assessments included: number of larvae present, size of larvae, strike size and larvae appearance. Larvae surviving at 72 hours were removed from the treated sheep and placed into a labelled plastic container and maintained in the laboratory insectary on mutton to determine if they could complete their life cycle. The strikes were then clipped out and treated with sodium tetraborate powder to eliminate the viability of the strike and minimise animal discomfort. Negative control strikes were resolved after the 24 hour assessment.

Both Investigational Veterinary Product (IVP) groups showed a significant effect on the strikes 24 hours post-implant until 20 weeks post-treatment (WAT).

The mean number of strikes controlled by both IVPs at 4 WAT was 13/16 at 24 hours, and both reached 16/16 by 72 hours post implant.

At 8 WAT the number of strikes controlled at 24 hours was 16/16 for both IVP 5 and IVP 6.

At 12 WAT, IVP 5 provided 16/16 strikes controlled at 24 hours, IVP 6 had controlled 15/16 of strikes. At 16 WAT IVP 5 and IVP 6 controlled 16/16 strikes by 72 hours post implant. At 20 WAT the efficacy of the IVPs declined to 13/16 at 72 hours.

The decline in efficacy continued at 24 WAT when for IVP 5 5/16 of strikes and for IVP 6 3/16 of the strikes were controlled at 72 hours.

Both IVP groups with uncontrolled strikes had significantly fewer larvae and smaller strikes than the untreated control group at 24 hours post-implanting.

There was no statistically significant difference between the efficacies in the treated groups at 72 hours at any time point during the trial. IVP 5 as well as IVP 6 showed a high level of efficacy until 16 WAT and the activity remained at moderate level until 20 WAT.

Where strikes were not completely controlled, IVP 5 and IVP 6 treatments demonstrated efficacy by restricting the size of the strikes and the number of larvae. Low numbers of larvae were collected at 4 and 12 WAT from uncontrolled strikes in Groups 1 and 2 and allowed to complete their life cycle on untreated mutton in the insectary. None of the larvae developed into pupal or adult stages.

The numbers of collected larvae as well as pupae increased at 20 WAT as the efficacy of the treatments declined with 36% (9) of larvae developing into pupae in Group 1 and 93% (28) in Group 2. However zero pupae developed into adult flies in any of the treatment groups.

At 24 WAT there were considerable numbers of larvae collected in all groups. In Group 1, 70% (71) larvae developed into pupae and 5% (5) adult flies emerged. In Group 2, 76% (93) larvae developed into pupae and 20% (25) adults emerged.

The data suggests that despite the fact the collected larvae appeared mostly unaffected and were allowed to complete their life cycle in an untreated environment their further development was generally inhibited as the pupal emergence of adult flies was low.

### Example 4: Evaluation of the efficacy of a dicyclanil spray-on formulation as a preventative treatment against fly strike in sheep caused by field strains of various Australian blowflies

### Formulation details

### Investigational Veterinary Product (IVP 7): Formulation Components

| **Formulation Components** | **g/L** |
|---|---|
| Purified Water | qs to 1 L |
| Morwet D 425 | 3.0 |
| Propylene Glycol | 75.98 |
| Polyoxyethylene Sorbitan Monooleate (Ecoteric T80) | 30 |
| Antifoam A | 0.85 |
| Antifoam C (Gensil 2030) | 0.65 |
| Disperse Scarlet 3610 | 0.30 |
| * Dicyclanil | 33.33 |
| Veegum | 5.8 |
| Kelzan S | 1.78 |
| Teric 200 | 22.4 |
| Gafac RE 610 | 5.6 |
| Formalin 37% | 0.30 |
| Sodium Hydroxide - 10% Solution | 0.40 |
| Sylgard 309 | 100 |

| | |
|---|---|
| Note: * Adjust amount of Dicyclanil based on purity of active material. IVP 7 was prepared by a process similar to that described for IVP 5 in Example 3. | |

### Results and discussion

Ten field studies were conducted over 9 months to evaluate the efficacy of IVP 7 as a preventative treatment against fly strike in sheep caused by field strains of various Australian blowflies. The studies were conducted in New South Wales, Victoria, South Australia and Western Australia on properties known to have been affected by fly strike in the past.

Ten sheep were weighed and treatment rates were based on the weight of the heaviest sheep from the two groups combined. Wool length was also measured on the 10 sheep at the shoulder, mid-back and hip regions prior to treatment. The formulation was applied with a treatment volume of 54 or 45 mL (30-105 mg/kg body weight) of the formulation was applied to the sheep by pour-on applicator (Simcro, NZ) with a spray on nozzle held 15-25 cm above the animal so as to deliver a 15 cm wide band. The formulation was applied in two bands along the back of the animal overlapping along the mid-line, and a third band on the crutch and rump areas of the animal.

The sheep at 9 sites were treated over a 3 week period. One site was a late entry into the study. The sheep were inspected for fly strike by the cooperating graziers on a regular basis. The sheep were inspected more often during favourable fly conditions. The Investigators visited the trial sites monthly to inspect the sheep for fly strike with the graziers and assess the skin and fleece reactions. Sheep blowfly surveys were also conducted at these times on the trial properties and neighbouring farms to provide information on fly pressure in the area. Daily minimum and maximum temperatures and humidity were recorded by data loggers placed in the paddocks with the sheep. Rainfall and wind speed greater than 30 km/hr were also recorded daily by the cooperating graziers.

The results from this study showed that only low numbers of sheep were struck in the IVP 7 group (7 animals) at any time during the field trial. Seven of the ten sites had no strikes recorded whilst two sites had three or less strikes recorded. At one site there were 4 strikes recorded in the IVP 7 group. This property experienced a period of wet weather followed by a fly wave accompanied with the sheep scouring and becoming very daggy. As a result 4 sheep in the IVP 7 group became fly struck in the crutch area on this study site.

Daggy wool on the treated animals compromises the efficacy of any preventative external treatment as the first instar larvae are able to survive in the dag only, protected from the treated wool and progress through the life cycle without being sufficiently exposed to the treatment immediately after hatching.

Fly strikes recorded in other mobs on the trial properties and in neighbouring properties indicated that there was sufficient fly pressure to test the efficacy of the IVP 7 product throughout the study period.

At 4 study sites the humid weather accompanied with the incidence of fly strikes in other mobs on the study sites and neighbouring properties arrived late in the study at approximately 5 months post treatments. Even under this fly pressure late in the study IVP 7 showed a satisfactory level of persistent protection from fly strike.

The results from this study showed that IVP 7 provided a high level of protection from fly strike on nine properties out of the total ten.

IVP 7 provided protection from field strains of the Australian blowflies for 24 weeks post treatment under favourable conditions for fly strike.

It was noted by the Investigators that IVP 7 was visible on the sheep for a longer period of time after the treatment application than any other commercially available formulation thus making it easier to distinguish sheep that had been treated from untreated animals.

There were no adverse skin or any other reactions or signs of wool damage recorded during the study.

It is to be understood that a reference herein to a prior art document does not constitute an admission that the document forms part of the common general knowledge in the art in Australia or in any other country.

In the claims which follow and in the preceding description of the invention, except where the context requires otherwise due to express language or necessary implication, the word "comprise" or variations such as "comprises" or "comprising" is used in an inclusive sense, i.e. to specify the presence of the stated features but not to preclude the presence or addition of further features in various embodiments of the invention.

## Claims

1. A topical formulation for use in the prevention or treatment of fly strike myiasis in sheep, wherein the formulation is topically applied as a pour-on or spray-on technique, the formulation comprising 20 g/L to 60 g/L dicyclanil in suspension, 20 g/L to 200 g/L of 3-(3-hydroxypropyl)- heptamethylsiloxane, ethoxylated, acetate surfactant and water.

2. The aqueous formulation for use according to claim 1, further comprising other surfactants selected from sorbitan esters, polyoxyalkylated sorbitan esters including polyoxyalkylated sorbitan fatty acid esters, polyoxyalkylated alkyl ethers, polyoxyalkylated fatty alcohols, polyoxyalkylated fatty acids, polyalkylene glycol esters, polyoxyalkylated vegetable oils, polyglycerol esters, copolymers of ethylene oxide and propylene oxide, fatty amine alkoxylates, alkylphenol alkoxylates, alkyl polysaccharides, polymeric surfactants, alkylnaphthalene sulphonates, C₁₂ to C₁₆ alcohol sulphates, C₁₂ alkoxypolyethanoxy sulphates, alkyl phosphates and phosphonates, alkylated alkylphenols, alkylphenol polyalkylate phosphate esters, benzalkonium, cetyltrimethylammonium chloride, cetyl pyridinium chloride, cetyl trimethylammonium bromide, tonzonium bromide and combinations thereof.

## Patentansprüche

1. Topische Formulierung zur Verwendung bei der Vorbeugung oder Behandlung von Fliegenmaden-Myiasis bei Schafen, wobei die Formulierung topisch mittels Aufgieß- oder Aufsprühtechnik aufgetragen wird, wobei die Formulierung 20 g/L bis 60 g/L Dicyclanil in Suspension, 20 g/L bis 200 g/L ethoxyliertes 3-(3-Hydroxypropyl)heptamethylsiloxan-Acetat-Tensid, und Wasser umfasst.

2. Wässrige Formulierung zur Verwendung gemäß Anspruch 1, ferner umfassend weitere Tenside, die aus Sorbitanestern, polyoxyalkylierten Sorbitanestern einschließlich polyoxyalkylierten Sorbitanfettsäureestern, polyoxyalkylierten Alkylethern, polyoxyalkylierten Fettalkoholen, polyoxyalkylierten Fettsäuren, Polyalkylenglykolestern, polyoxyalkylierten Pflanzenölen, Polyglycerinestern, Copolymeren von Ethylenoxid und Propylenoxid, Fettaminalkoxylaten, Alkylphenolalkoxylaten, Alkylpolysacchariden, polymeren Tensiden, Alkylnaphthalinsulfonaten, C₁₂- bis C₁₆₋ Alkoholsulfaten, C₁₂-Alkoxypolyethanoxysulfaten, Alkylphosphaten und - phosphonaten, alkylierten Alkylphenolen, Alkylphenolpolyalkylatphosphatestern, Benzalkonium, Cetyltrimethylammoniumchlorid, Cetylpyridiniumchlorid, Cetyltrimethylammoniumbromid, Tonzoniumbromid und Kombination davon ausgewählt sind.

## Revendications

1. Formulation topique pour une utilisation dans la prévention ou le traitement de la myase de mouche chez un mouton, la formulation étant appliquée de manière topique par une technique transcutanée ou par pulvérisation, la formulation comprenant 20 g/L à 60 g/L de dicyclanil en suspension, 20 g/L à 200 g/L de 3-(3-hydroxypropyl)-heptaméthylsiloxane, éthoxylé, un tensioactif de type acétate et de l'eau.

2. Formulation aqueuse pour une utilisation selon la revendication 1, comprenant en outre d'autres tensioactifs choisis parmi des esters de sorbitane, des esters de sorbitane polyoxyalkylés y compris des esters d'acide gras de sorbitane polyoxyalkylés, des alkyléthers polyoxyalkylés, des alcools gras polyoxyalkylés, des acides gras polyoxyalkylés, des esters de polyalkylèneglycol, des huiles végétales polyoxyalkylées, des esters de polyglycérol, des copolymères d'oxyde d'éthylène et d'oxyde de propylène, des alcoxylates d'amine grasse, des alcoxylates d'alkylphénol, des polysaccharides d'alkyle, des tensioactifs polymériques, des alkylnaphtalènesulfonates, des alcoolsulfates en C₁₂₋₁₆, des alcoxypolyéthanoxysulfates en C₁₂, des phosphates et des phosphonates d'alkyle, des alkylphénols alkylés, des esters de phosphate d'alkylphénol polyalkylate, un benzalkonium, le chlorure de cétyltriméthylammonium, le chlorure de cétylpyridinium, le bromure de cétyltriméthylammonium, le bromure de tonzonium et des combinaisons correspondantes.
